(19) Europäisches Patentamt
European Patent Office
Office européen des brevets

(11) **EP 3 420 903 B1**

(12) **EUROPÄISCHE PATENTSCHRIFT**

(45) Veröffentlichungstag und Bekanntmachung des Hinweises auf die Patenterteilung:
**23.10.2019   Patentblatt 2019/43**

(51) Int Cl.:
***A61B 6/00*** ***(2006.01)***

(21) Anmeldenummer: **17178792.2**

(22) Anmeldetag: **29.06.2017**

(54) **VISUALISIEREN ZUMINDEST EINER KENNGRÖSSE**

VISUALISATION OF AT LEAST ONE INDICATOR

VISUALISATION D'AU MOINS UN PARAMÈTRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.01.2019   Patentblatt 2019/01**

(73) Patentinhaber: **Siemens Healthcare GmbH
91052 Erlangen (DE)**

(72) Erfinder: **Rahn, Norbert
91301 Forchheim (DE)**

(56) Entgegenhaltungen:
**EP-A1- 3 128 481          WO-A1-2010/041201
WO-A1-2016/145010     DE-A1-102010 022 791
US-A1- 2015 112 182**

EP 3 420 903 B1

**Beschreibung**

Die Erfindung betrifft ein Verfahren zum Visualisieren zumindest einer Kenngröße eines Patienten bei einer angiographischen Messung mittels eines C-Bogen-Röntgengeräts, das zugehörige C-Bogen-Röntgengerät und ein zugehöriges Computerprogrammprodukt.

Ein angiographisches Bild, welches mit einer C-Bogen-Angulation mittels eines C-Bogen-Röntgengeräts in einem kardiologischen Katheterlabor aufgenommen wurde, weist typischerweise eine zweidimensionale (2D) Abbildung eines Gefäß, insbesondere einer kontrastierten Koronararterie, auf. Dabei beschreibt die C-Bogen-Angulation insbesondere eine Winkelkonfiguration des C-Bogen-Röntgengeräts. Abhängig von der kontrastierten Koronararterie und von der C-Bogen-Angulation kann das angiographische Bild eine Gefäßverkürzung und/oder eine Gefäßüberlappung der kontrastierten Koronararterie aufweisen. Typischerweise ist in diesem Fall aber kein Hinweis darauf vorhanden, dass die kontrastierte Koronararterie verkürzt bzw. überlappt dargestellt ist.

Die Gefäßverkürzung und/oder die Gefäßüberlappung können zu Fehlinterpretationen durch einen Nutzer oder einen Arzt führen, weil beispielsweise wegen der Gefäßverkürzung eine Quantifizierung von einer Gefäßlänge oder einem Gefäßdurchmesser der kontrastierten Koronararterie in dem angiographischen Bild nicht möglich ist. Daher kann beispielsweise die Gefäßlänge einer zu therapierenden Gefäß-Stenose wegen der Gefäßverkürzung unterschätzt werden. Daraus kann folgen, dass im Fall einer Stent-Implantation ein Implantat mit unzureichender Länge ausgewählt wird. Deshalb wird das zu therapierende Gefäß typischerweise mit verschiedenen C-Bogen-Angulationen erfasst bzw. aufgenommen, um dadurch einen besseren räumlichen Eindruck über das zu therapierende Gefäß zu gewinnen. Hierfür ist allerdings Intuition und Erfahrung des Nutzers/des Arztes nötig, damit gewährleistet ist, dass zumindest ein angiographisches 2D-Bild von allen angiographischen Bildern, welche mit verschiedenen C-Bogen-Angulationen generiert wurden, das zu therapierende Gefäß ohne Gefäßverkürzung, insbesondere verkürzungsfrei, aufweist.

In der DE 10 2015 202 082 A1, der DE 10 2014 202 013 A1 und der DE 10 2012 208 850 A1 sind C-Bogen-Röntgengeräte beschrieben, mittels welcher angiographische Messungen durchgeführt werden können. Ein weiteres Beispiel ist in EP-A-3 128 481 gezeigt, welches mindestens ein Bildinformationssystem bereitstellt und ein 2D-Bild mittels eines C-Bogen-Röntgengeräts erfasst.

Der Erfindung liegt die Aufgabe zu Grunde, ein verbessertes Verfahren zum Visualisieren zumindest einer Kenngröße eines Patienten bei einer angiographischen Messung mittels eines C-Bogen-Röntgengeräts, das zugehörige C-Bogen-Röntgengerät und ein zugehöriges Computerprogrammprodukt anzugeben.

Die Aufgabe wird durch die Merkmale der unabhängigen Ansprüche gelöst. Vorteilhafte Weiterbildungen sind in den abhängigen Ansprüchen angegeben.

Nachstehend wird die erfindungsgemäße Lösung der Aufgabe sowohl in Bezug auf das beanspruchte C-Bogen-Röntgengerät als auch auf das Verfahren zum Visualisieren zumindest einer Kenngröße eines Patienten bei einer angiographischen Messung mittels eines C-Bogen-Röntgengeräts sowie auf das zugehörige Computerprogrammprodukt beschrieben. Hierbei erwähnte Merkmale, Vorteile oder alternative Ausführungsformen sind ebenso auf die anderen beanspruchten Gegenstände zu übertragen und umgekehrt. Mit anderen Worten können die gegenständlichen Ansprüche (die beispielsweise auf ein C-Bogen-Röntgengerät gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Das erfindungsgemäße Verfahren zum Visualisieren zumindest einer Kenngröße eines Patienten bei einer angiographischen Messung mittels eines C-Bogen-Röntgengeräts umfasst folgende Schritte:

- Bereitstellen eines Bildinformationssystems,
- Erfassen zumindest eines 2D-Bildes des Patienten mittels des C-Bogen-Röntgengeräts,
- Bestimmen zumindest eines 3D-Referenz-Bildes durch Anwenden des Bildinformationssystems auf das zumindest eine 2D-Bild,
- Ermitteln zumindest einer Kenngröße, welche zumindest ein Gefäß des Patienten beschreibt, in dem zumindest einen 3D-Referenz-Bild und
- Visualisieren der zumindest einen Kenngröße in dem zumindest einen 2D-Bild.

Das Bereitstellen des Bildinformationssystems kann umfassen, dass das Bildinformationssystem in einer Klinik oder in einem Rechenzentrum von einem Hersteller des Bildinformationssystems oder einem Personal der Klinik oder einem Personal des Rechenzentrums eingerichtet wird. Das Einrichten oder Konfigurieren des Bildinformationssystems umfasst üblicherweise eine Konfiguration des Bildinformationssystems mit geeigneter Hardware (z.B. Arbeitsspeicher, Festplatte, Ein-/Ausgabe-Schnittstellen, Monitor, Eingabegeräte, Speichereinheit, Prozessor, etc.) und/oder eine Installation von entsprechender Software, beispielsweise eines Betriebssystems und/oder eines Computerprogramm-Produkts zum Bestimmen des zumindest einen 3D-Referenz-Bildes durch Anwenden des Bildinformationssystems auf das zumindest eine 2D-Bild.

Vorzugsweise ist das Bildinformationssystem direkt mit dem C-Bogen-Röntgengerät, insbesondere einer Speichereinheit des C-Bogen-Röntgengeräts, verbunden. Dafür können das Bildinformationssystem und/oder das C-Bogen-Röntgengerät und/oder die Speichereinheit des C-Bogen-Röntgengeräts geeignete Schnittstellen aufweisen, die beispielsweise einen Austausch insbesondere des zumindest einen 2D-Bildes, des zumindest einen 3D-Referenz-Bildes und des zumindest einen 2D-Bildes, in welchem die zumindest eine Kenngröße visualisiert ist, ermöglichen. Das Bildinformationssystem kann in dem Rechenzentrum vorliegen und insbesondere mittels einer geeigneten Schnittstelle mit dem C-Bogen-Röntgengerät verbunden sein. Alternativ kann das Bildinformationssystem als Komponente des C-Bogen-Röntgengeräts ausgebildet sein.

Vorzugsweise umfasst das Erfassen des zumindest einen 2D-Bildes eine Injektion eines Kontrastmittelbolus mittels einer Kontrastmittelinjektionseinheit des C-Bogen-Röntgengeräts. Üblicherweise wird ein Jod-haltiger Kontrastmittelbolus injiziert. Typischerweise wird ein nicht-kontrastverstärktes Bild vor der Injektion des Kontrastmittelbolus erfasst und ein angiographisches Bild nach der Injektion des Kontrastmittelbolus erfasst. Das zumindest eine 2D-Bild kann in diesem Fall einer Subtraktion des nicht-kontrastverstärkten 2D-Bildes von dem angiographischen 2D-Bild entsprechen. Alternativ kann das Erfassen des zumindest einen 2D-Bildes ein Laden des 2D-Bildes insbesondere aus der Speichereinheit des C-Bogen-Röntgengeräts beschreiben.

Das zumindest eine 2D-Bild weist vorzugweise das zumindest eine Gefäß auf. In einer bevorzugten Ausführung weist das zumindest eine 2D-Bild lediglich das zumindest eine Gefäß auf. Das zumindest eine 2D-Bild kann eine Gefäßstruktur des Patienten, insbesondere mehrere Gefäße des Patienten aufweisen. Das zumindest eine Gefäß kann eine arterielle und/oder eine venöse Struktur einer Gefäßstruktur des Patienten aufweisen. Das zumindest eine Gefäß umfasst beispielsweise entweder ein Gefäßsegment oder mehrere Gefäße der Gefäßstruktur. Beispielsweise kann das zumindest eine Gefäß einer Körperregion Herz zugeordnet werden. Das zumindest eine Gefäß kann eine Koronararterie (z.B. RCA = Arteria coronaria dextra) und/oder kapillare Gefäße aufweisen.

Die zumindest eine Kenngröße beschreibt das zumindest eine Gefäß des Patienten. Die zumindest eine Kenngröße ist daher beispielsweise für einen Nutzer des C-Bogen-Röntgengeräts oder einen Arzt besonders relevant, weil das zumindest eine Gefäß des Patienten während der angiographischen Messung untersucht oder therapiert werden soll. Beispielsweise führt der Arzt vor, während oder nach der angiographischen Messung einen chirurgischen oder interventionellen Eingriff, welcher das zumindest eine Gefäß betrifft, durch.

Das Anwenden des Bildinformationssystems auf das zumindest eine 2D-Bild umfasst üblicherweise ein Bereitstellen des erfassten zumindest einen 2D-Bildes als Eingabeparameter für das Bildinformationssystem. Das Bildinformationssystem weist vorzugsweise geeignete Mittel zu einer Eingabe des zumindest einen 2D-Bildes als den Eingabeparameter auf. Die dafür geeigneten Mittel können Schnittstellen für ein Netzwerk zwischen dem Bildinformationssystem und dem C-Bogen-Röntgengerät oder ein DVD-Laufwerk aufweisen.

Insbesondere ist in dem Bildinformationssystem eine Bildmenge aus mehreren 3D-Bildern hinterlegt bzw. abgespeichert. So weist das Bildinformationssystem insbesondere Mittel zum Auswählen und Abrufen des zumindest einen 3D-Referenz-Bildes aus der Bildmenge der mehreren 3D-Bilder auf. Beispielsweise kann der Hersteller des Bildinformationssystems oder das Personal in der Klinik die Bildmenge auf das Bildinformationssystem aufgespielt haben. Das ist insbesondere vorteilhaft, weil der Patient nicht vor der angiographischen Messung für eine Aufnahme des zumindest einen 3D-Referenz-Bildes mittels des C-Bogen-Röntgengeräts oder mittels eines Computertomographen gemessen werden muss, sondern dass die Bildmenge aus mehreren 3D-Bildern, aus welchen üblicherweise das zumindest eine 3D-Referenz-Bild ausgewählt wird, typischerweise bereits vor dem Erfassen des zumindest einen 2D-Bildes vorliegt.

Die Bildmenge kann alternativ oder zusätzlich zu den mehreren 3D-Bildern ein 3D-Modell des zumindest einen Gefäßes umfassen. Ein erweitertes 3D-Modell kann einer idealisierten oder modellierten Variante des zumindest einen Gefäßes entsprechen. Das erweiterte 3D-Modell kann aus einer Vielzahl von verschiedenen 3D-Bildern von verschiedenen Patienten gebildet worden sein. Die mehreren 3D-Bilder werden insbesondere aus Messdaten, welche beispielsweise mittels eines Computertomographen generiert worden sind, rekonstruiert.

Üblicherweise zeigt die Bildmenge das Herz zu einer bestimmten Herzphase und eine Information über die Herzphase kann in einer Beschreibung, insbesondere in einem Header gemäß dem DICOM-Standard, des jeweiligen 3D-Bildes gespeichert sein. Abhängig von der Herzphase verändert sich üblicherweise das zumindest eine Gefäß, beispielsweise gemäß einer Kontraktion des Herzens. Vorzugsweise weist die Bildmenge mehrere 3D-Bilder zu je unterschiedlichen Herzphasen, insbesondere über einen gesamten Herzzyklus, auf. Die Bildmenge kann also insbesondere 3D-Bilder-Szenen bzw. vierdimensionale (4D) Bilder aufweisen, wobei die 4D-Bilder zeitaufgelöste 3D-Bilder umfassen. Die zeitaufgelösten 3D-Bilder können insbesondere wenigstens einer Herzphase zugeordnet werden.

Das Bildinformationssystem verarbeitet insbesondere das zumindest eine 2D-Bild derart, dass das zumindest eine 3D-Referenz-Bild bestimmt wird. Das zumindest eine 3D-Referenz-Bild wird vorzugsweise derart bestimmt, dass eine möglichst hohe Ähnlichkeit oder ein möglichst großer Korrelationsfaktor zwischen dem zumindest einen 2D-Bild und einem Bildinhalt des zumindest einen 3D-Referenz-Bild vorliegt. Insbesondere wird das zumindest eine 2D-Bild mit allen 3D-Bildern der Bildmenge mittels der Ähnlichkeitsanalyse oder der Korrelation verglichen. Es wird insbesondere dasjenige 3D-Bild aus der Bildmenge der mehreren 3D-Bilder des Bildinformationssystems als 3D-Referenz-Bild ausgewählt, dessen Bildinhalt den größten Korrelationsfaktor mit dem zumindest einen 2D-Bild aufweist. Alternativ oder zusätzlich wird insbesondere das zumindest eine 2D-Bild mit einem Bildinhalt der mehreren 3D-Bildern der Bildmenge registriert. Das Bestimmen des zumindest einen 3D-Referenz-Bildes durch Anwenden des Bildinformationssystems auf das zumindest eine 2D-Bild kann eine mathematische Korrelation und/oder eine mehrdimensionale Interpolation und/oder ein Laden des zumindest einen 3D-Referenz-Bildes aus einem Speicher des Bildinformationssystem umfassen. Letzteres ist insofern möglich, wenn das zumindest eine 3D-Referenz-Bild bereits zu einem früheren Zeitpunkt bestimmt worden ist. Das Laden kann insbesondere vorteilhaft sein, wenn das Bestimmen des zumindest einen 3D-Referenz-Bildes durch das Anwenden des Bildinformationssystems auf das zumindest eine 2D-Bild rechenintensiv und/oder zeitlich aufwändig ist.

Das Bestimmen des zumindest einen 3D-Referenz-Bildes durch das Anwenden des Bildinformationssystem auf das zumindest eine 2D-Bild ist üblicherweise ein rechenintensiver Vorgang, weil insbesondere eine NxN-Matrix des zumindest einen 2D-Bildes in einer MxMxM-Matrix der mehreren 3D-Bilder der Bildmenge des Bildinformationssystems gefunden werden soll. Üblicherweise wird das zumindest eine 2D-Bild Bild-für-Bild mit den mehreren 3D-Bildern der Bildmenge verglichen. Typischerweise wird mittels einer multi-planaren Rekonstruktion aus der MxMxM-Matrix eine M'xM'-Matrix berechnet, weil eine Korrelation zwischen der NxN-Matrix mit der M'xM'-Matrix weniger rechenintensiv ist als eine Korrelation der NxN-Matrix mit der MxMxM-Matrix. Die multi-planare Rekonstruktion der M'xM'-Matrix besitzt typischerweise unendliche viele Lösungen. Idealerweise wird die multi-planare Rekonstruktion mit einem bestimmten Winkel, insbesondere der C-Bogen-Angulation des C-Bogen-Röntgengeräts, vorgegeben, wodurch die Bildmenge für die Korrelation oder die Ähnlichkeitsanalyse oder die Registrierung eingeschränkt wird.

Vorteilhafterweise ist das Bestimmen des zumindest einen 3D-Referenz-Bildes durch das Anwenden des Bildinformationssystems auf das zumindest eine 2D-Bild eindeutig. Das bedeutet in anderen Worten, für genau eine Eingabe existiert genau ein Ergebnis im Bildinformationssystem.

Typischerweise ist das Bestimmen des zumindest einen 3D-Referenz-Bildes durch das Anwenden des Bildinformationssystem auf das zumindest eine 2D-Bild desto schneller, exakter und/oder eindeutiger, je mehr Bildinformationen das zumindest eine 2D-Bild aufweist. Die Bildinformationen können einem Bildinhalt des zumindest einen 2D-Bildes und/oder Beschreibungsparametern, die in dem Header des zumindest einen 2D-Bildes vorliegen, entsprechen. Desto mehr der Bildinformationen vorliegen, umso kleiner wird insbesondere die Bildmenge für die Korrelation oder die Ähnlichkeitsanalyse. Je kleiner die Bildmenge ist, insbesondere desto schneller wird das zumindest eine 3D-Referenz-Bild bestimmt. Die Korrelation oder die Ähnlichkeit kann durch zusätzliche Rahmenbedingungen, die aus dem zumindest einen 2D-Bild hervorgehen, verbessert werden.

Das Ermitteln der zumindest einen Kenngröße umfasst vorzugsweise ein Anwenden eines Algorithmus, welcher bevorzugt derart ausgebildet ist, dass die zumindest eine Kenngröße, die das erfasste zumindest eine 2D-Bild nicht aufweist, aus dem 3D-Referenz-Bild ermittelt werden kann. Die zumindest eine Kenngröße umfasst vorzugweise bildliche Tiefeninformationen des zumindest einen 3D-Referenz-Bildes, die das zumindest eine 2D-Bild nicht unmittelbar aufweist.

Die zumindest eine Kenngröße wird vorzugsweise derart in dem zumindest einen 2D-Bild visualisiert, dass das zumindest eine 2D-Bild zumindest einen Teil der bildlichen Tiefeninformationen des zumindest einen 3D-Referenz-Bildes aufweist. Dadurch kann das zumindest eine 2D-Bild einen räumlichen Eindruck aufweisen.

Eine Ausführungsform sieht vor, dass die zumindest eine Kenngröße eine Gefäßverkürzung des zumindest einen Gefäßes beschreibt. Die zumindest eine Kenngröße kann ein Maß für eine Ausprägung der Gefäßverkürzung, oder für eine Wahrscheinlichkeit eines Auftretens der Gefäßverkürzung sein. Die zumindest eine Kenngröße weist in diesem Fall vorzugsweise Informationen darüber auf, inwiefern das zumindest eine Gefäß in dem zumindest einen 2D-Bild verkürzt dargestellt wird. Vorzugsweise wird die Gefäßverkürzung desto deutlicher durch die zumindest eine Kenngröße gekennzeichnet, je verkürzter das zumindest eine Gefäß in dem zumindest einen 2D-Bild dargestellt wird.

Eine Ausführungsform sieht vor, dass die zumindest eine Kenngröße eine Gefäßüberlappung des zumindest einen Gefäßes beschreibt. Die zumindest eine Kenngröße weist in diesem Fall vorzugsweise Informationen darüber auf, inwiefern das zumindest eine Gefäß in dem zumindest einen 2D-Bild von einem anderen Gefäß und/oder von einem Teil des zumindest einen Gefäßes, insbesondere teilweise, überlappt dargestellt ist.

Eine Ausführungsform sieht vor, dass dem zumindest einen 2D-Bild zumindest ein Meta-Parameter zugeordnet ist, wobei der zumindest eine Meta-Parameter beim Bestimmen des zumindest einen 3D-Referenz-Bildes verwendet wird und der zumindest eine Meta-Parameter mindestens einen Parameter der folgenden Liste aufweist:

- eine Meta-Information über das Erfassen des zumindest einen 2D-Bildes mittels des C-Bogen-Röntgengeräts,
- eine Gefäßbezeichnung des zumindest einen Gefäßes und
- eine Herzphase des Patienten.

Grundsätzlich ist es denkbar, dass die Meta-Information über das Erfassen des zumindest einen 2D-Bildes mittels des C-Bogen-Röntgengeräts und/oder die Herzphase des Patienten automatisch dem 2D-Bild zugeordnet, insbesondere in dem Header des zumindest einen 2D-Bildes gespeichert, wird. Die Meta-Information weist insbesondere eine Konfiguration des C-Bogen-Röntgengeräts bei der angiographischen Messung auf und wird online, insbesondere in Echtzeit, erfasst und dem zumindest einen 2D-Bild zugeordnet.

Die Herzphase des Patienten wird beispielsweise mit einem Echo-Kardiographen online, insbesondere während der angiographischen Messung in Echtzeit, erfasst, wobei der Echo-Kardiograph über eine Schnittstelle mit dem C-Bogen-Röntgengerät verbunden ist. Die Herzphase wird dem zumindest einen 2D-Bild zugeordnet, insbesondere in dem Header des zumindest einen 2D-Bildes gespeichert. Unter Berücksichtigung der Herzphase kann das Bestimmen des zumindest einen SD-Referenz-Bildes dadurch verbessert werden, dass insbesondere in der Bildmenge für die Ähnlichkeitsanalyse nur diejenigen 3D- Bilder mit der exakten oder zumindest ähnlichen Herzphase des zumindest einen 2D-Bildes verbleiben. Grundsätzlich ist es auch denkbar, dass das zumindest eine 2D-Bild gemäß der Herzphase ausgewählt wird und dementsprechend das zumindest eine 3D-Referenz-Bild bestimmt wird. Des Weiteren kann das zumindest eine 2D-Bild mehrere Herzphasen umfassen, so dass durch das Anwenden des Bildinformationssystems auf das zumindest eine 2D-Bild mit mehreren Herzphasen das zumindest eine 3D-Referenz-Bild mit mehreren Herzphasen bestimmt wird. Die mehreren Herzphasen können insbesondere einen Herzzyklus bilden.

Weiterhin kann der zumindest eine Meta-Parameter die Gefäßbezeichnung des zumindest einen Gefäßes aufweisen, z.B. RCA. Die Gefäßbezeichnung des zumindest einen Gefäßes kann vorzugsweise durch einen Nutzer auf einem Monitor des C-Bogen-Röntgengeräts dem zumindest einen 2D-Bild zugeordnet werden, wobei der Meta-Parameter insbesondere in dem Header des zumindest einen 2D-Bildes gespeichert wird. Der Nutzer kann auf dem Monitor des C-Bogen-Röntgengeräts mit einer graphischen Benutzeroberfläche interagieren. Üblicherweise weist das C-Bogen-Röntgengerät dafür ein Eingabegerät wie z.B. eine Maus oder eine Tastatur auf. Alternativ oder zusätzlich ist es denkbar, dass mittels Landmarkenerkennung dem zumindest einen 2D-Bild die Gefäßbezeichnung automatisch zugeordnet wird. Unter Berücksichtigung der Gefäßbezeichnung kann das Bestimmen des zumindest einen 3D-Referenz-Bildes dadurch verbessert werden, dass die Bildmenge der 3D-Bilder des Bildinformationssystems auf diejenigen 3D-Bilder, welche das Gefäß mit der passenden Gefäßbezeichnung aufweist, eingeschränkt wird. Beispielsweise werden nur diejenigen 3D-Bilder berücksichtigt, welchen ein Gefäß mit der Gefäßbezeichnung des zumindest einen Gefäßes zugeordnet ist.

Eine Ausführungsform sieht vor, dass der zumindest eine Meta-Parameter die Gefäßbezeichnung des zumindest einen Gefäßes aufweist und wobei die Gefäßbezeichnung mindestens einen Parameter der folgenden Liste aufweist:

- einen Gefäßdurchmesser des zumindest einen Gefäßes,
- eine maximale Krümmung des zumindest einen Gefäßes und
- eine Länge des zumindest einen Gefäßes.

Der Gefäßdurchmesser, die maximale Krümmung und die Länge des zumindest einen Gefäßes können einer Gruppe von Parametern zugeordnet werden, welche semantische Informationen über das Gefäß aufweist. Das zumindest eine Gefäß wird in diesem Fall mit deskriptiven Merkmalen beschrieben bzw. identifiziert. Das Bestimmen des zumindest einen 3D-Referenz-Bildes durch das Anwenden des Bildinformationssystems auf das zumindest eine 2D-Bild kann gemäß den deskriptiven Merkmalen erfolgen. Der Gefäßdurchmesser, die maximale Krümmung und/oder die Länge des zumindest einen Gefäßes können beispielsweise durch den Nutzer oder automatisch in dem zumindest einen 2D-Bild erfasst werden. Der Gefäßdurchmesser, die maximale Krümmung und/oder die Länge des zumindest einen Gefäßes können insbesondere aus einer medizinischen Fachliteratur oder gemäß Standardwerte gewählt sein oder insbesondere aus Metriken, die mit einer Vielzahl von Trainingsgefäßen trainiert wurden, gebildet sein. Vorzugsweise sind den 3D-Bildern der Bildmenge die jeweiligen deskriptiven Merkmale des Bildinhalts der 3D-Bilder zugeordnet, so dass durch das Anwenden des Bildinformationssystems auf das zumindest eine 2D-Bild gemäß den deskriptiven Merkmalen insbesondere die Bildmenge eingeschränkt wird.

Eine Ausführungsform sieht vor, dass der zumindest eine Meta-Parameter die Meta-Information über das Erfassen des zumindest einen 2D-Bildes mittels des C-Bogen-Röntgengeräts aufweist und wobei die Meta-Information mindestens einen Parameter der folgenden Liste aufweist:

- eine räumliche Lage des C-Bogen-Röntgengeräts,
- einen Abstand zwischen einem Röntgenstrahler des C-Bogen-Röntgengeräts und einem Röntgendetektor des C-Bogen-Röntgengeräts und
- eine räumliche Lage des Patiententisches.

Das C-Bogen-Röntgengerät weist vorzugweise zumindest ein Koordinatensystem zur Angabe der räumlichen Lage des C-Bogen-Röntgengeräts und der räumlichen Lage des Patiententisches auf. Die räumliche Lage des C-Bogen-Röntgengeräts weist insbesondere eine Position in dem zumindest einen Koordinatensystem, eine Orientierung des C-Bogen-Röntgengeräts und eine Konfiguration für die Position und die Orientierung auf. Zusätzlich kann die räumliche des C-Bogen-Röntgengeräts die Konfiguration aufweisen, wie die Position des C-Bogen-Röntgengeräts und Orientierung des C-Bogen-Röntgengeräts erreicht worden ist, beispielsweise durch ein Einfahren oder Ausfahren einer Teleskopeinheit des C-Bogen-Röntgengeräts oder durch ein Verdrehen eines Drehgelenks des C-Bogen-Röntgengeräts.

Mittels der räumlichen Lage des C-Bogen-Röntgengeräts kann insbesondere eine C-Bogen-Angulation ermittelt werden, wobei die C-Bogen-Angulation insbesondere für das Bestimmen des zumindest einen 3D-Referenz-Bildes durch das Anwenden des Bildinformationssystems auf das zumindest eine 2D-Bild relevant ist, weil durch die Vorgabe der C-Bogen-Angulation der Winkel für die multi-planare Rekonstruktion vorgegeben wird, wodurch das Bestimmen des zumindest einen 3D-Referenz-Bildes wesentlich beschleunigt wird. Durch die Vorgabe der C-Bogen-Angulation wird die Bildmenge der 3D-Bilder insofern eingeschränkt, weil lediglich die 3D-Bilder ausgewählt werden können, welchen die C-Bogen-Angulation zugeordnet ist. Beispielsweise weist die räumliche Lage einen angularen Winkel des C-Bogen-Röntgengeräts und einen orbitalen Winkel des C-Bogen-Röntgengeräts, insbesondere die C-Bogen-Angulation des C-Bogen-Röntgengeräts, auf.

Der Abstand zwischen dem Röntgenstrahler und dem Röntgendetektor gibt üblicherweise ein Maß für die Skalierung des zumindest einen 2D-Bildes vor. In der Optik wird das Maß für die Skalierung üblicherweise Zoom oder Zoom-Faktor genannt. Vorzugweise werden beim Bestimmen des 3D-Referenz-Bildes desto mehr 3D-Bilder mit eher kleineren Gefäßen berücksichtigt, je geringer der Abstand zwischen dem Röntgenstrahler und dem Röntgendetektor ist.

Mittels des zumindest einen Meta-Parameters kann vorteilhafterweise die Bildmenge der 3D-Bilder, deren Umfang üblicherweise direkt mit einer rechnerischen Komplexität des Bestimmens des zumindest einen 3D-Referenz-Bildes skaliert, eingeschränkt werden. Insbesondere ist es denkbar, dass für den zumindest einen Meta-Parameter ein Toleranzbereich vorgegeben wir. Der Toleranzbereich beschreibt insbesondere eine Rahmenbedingung für die Ähnlichkeitsanalyse oder die Korrelation oder die Registrierung. Die Bildmenge ist insofern desto eingeschränkter, je kleiner der Toleranzbereich ist.

Das Bestimmen des zumindest einen 3D-Referenz-Bildes durch das Anwenden des Bildinformationssystems auf das zumindest eine 2D-Bild kann ein Modifizieren des zumindest einen 2D-Bildes durch ein Bewegungsfeld umfassen. Das Bewegungsfeld entspricht insbesondere einer mathematischen Operation, gemäß welcher das zumindest eine 2D-Bild verändert wird. Beispielsweise kann das Bewegungsfeld eine Herzphasen-Transformation aufweisen. Durch das Modifizieren des zumindest einen 2D-Bildes einer ersten Herzphase durch das Bewegungsfeld kann beispielsweise basierend auf dem zumindest einen 2D-Bild ein weiteres 2D-Bild bei einer zweiten Herzphase berechnet werden. Durch das Modifizieren gemäß dem Bewegungsfeld kann daher das zumindest eine 2D-Bild zu unterschiedlichen Herzphasen simuliert werden. Die 3D-Bilder der Bildmenge können üblicherweise gemäß dem Bewegungsfeld angepasst werden, wodurch insbesondere ein höherer Korrelationsfaktor gefunden wird als wenn die 3D-Bilder nicht angepasst werden.

Des Weiteren ist es denkbar, dass mittels geeigneter Sensoren, insbesondere einer Kamera oder eines Atemgurts, eine Atembewegung des Patienten erfasst wird. Gemäß der Atembewegung des Patienten kann das Bewegungsfeld berechnet werden, wobei das zumindest eine 2D-Bild durch das Bewegungsfeld modifiziert wird. Das Bewegungsfeld kann sowohl gemäß der Atembewegung des Patienten als auch gemäß der Herzphase des Patienten berechnet werden.

Eine Ausführungsform sieht vor, dass das Bestimmen des zumindest einen 3D-Referenz-Bildes durch das Anwenden des Bildinformationssystems auf das zumindest eine 2D-Bild umfasst:

- Segmentieren des zumindest einen 2D-Bildes gemäß dem zumindest einen Gefäß und
- Anwenden des Bildinformationssystems auf das zumindest eine segmentierte 2D-Bild.

Mittels des Segmentierens des zumindest einen 2D-Bildes gemäß dem zumindest einen Gefäß kann vorzugsweise ein Bereich des zumindest einen 2D-Bildes, bei dem das Visualisieren der zumindest einen Kenngröße besonders sinnvoll ist, hervorgehoben werden. Üblicherweise wird die zumindest eine Kenngröße nur gemäß dem zumindest einen Gefäß visualisiert. Der Bereich ohne das zumindest eine Gefäß ist üblicherweise von niedrigem oder keinem Interesse für den Nutzer.

Insbesondere das grauwert- oder modellbasierte Segmentieren des zumindest einen 2D-Bildes kann manuell, semi-automatisch oder vollautomatisch durchgeführt werden. Wenn das zumindest eine 2D-Bild gemäß dem zumindest einen Gefäß segmentiert wird, verbleibt insbesondere lediglich für das Bestimmen des zumindest einen 3D-Referenz-Bildes relevante Bildinformation in dem zumindest einen 2D-Bild. Beispielsweise kann das zumindest eine Gefäß in einer Mittellinie abgebildet sein. Das Segmentieren kann in einer Binärmaske des zumindest einen 2D-Bildes resultieren. Alternativ oder zusätzlich kann eine Kontur des zumindest einen Gefäßes durch das Segmentieren ermittelt werden.

Grundsätzlich ist es denkbar, dass die mehreren 3D-Bilder der Bildmenge vor dem Bestimmen des zumindest einen SD-Referenz-Bildes analog zu dem zumindest einen 2D-Bild segmentiert werden. In das Bestimmen des zumindest einen 3D-Referenz-Bildes geht insbesondere lediglich der segmentierte Bildinhalt ein. In einer bevorzugten Ausführung wird durch das Anwenden des Bildinformationssystems auf das zumindest eine segmentierte 2D-Bild das 3D-Referenz-Bild schneller bestimmt als durch das Anwenden des Bildinformationssystem auf das 2D-Bild ohne vorherige Segmentierung. Dieser Effekt erfolgt üblicherweise dadurch, dass durch das Segmentieren des zumindest einen 2D-Bildes weniger irrelevanter Bildinhalt des segmentierten 2D-Bildes mit den 3D-Bildern der Bildmenge korreliert oder registriert wird. Vorteilhafterweise ist die Korrelation oder die Ähnlichkeitsanalyse oder die Registrierung desto schneller, je weniger irrelevanten Bildinhalt wie beispielsweise Hintergrund das segmentierte 2D-Bild aufweist.

Eine Ausführungsform sieht vor, dass das Bestimmen des zumindest einen 3D-Referenz-Bildes durch das Anwenden des Bildinformationssystems auf das zumindest eine 2D-Bild ein Ermitteln eines 2D-Referenz-Bildes, wobei das 2D-Referenz-Bild dem zumindest einen 3D-Referenz-Bild zugeordnet ist, umfasst.

Das Bildinformationssystem kann insbesondere eine weitere 2D-Bildmenge an 2D-Bildern umfassen, wobei das zumindest eine 2D-Referenz-Bild aus der weiteren 2D-Bildmenge bestimmt wird. Das Ermitteln des 2D-Referenz-Bildes erfolgt vorzugsweise automatisch durch das Anwenden des Bildinformationssystems auf das zumindest eine 2D-Bild, insbesondere gemäß den gleichen Algorithmen, die für das Bestimmen des zumindest einen 3D-Referenz-Bildes durch das Anwenden des Bildinformationssystems auf das zumindest eine 2D-Bild verwendet werden. Insbesondere werden die mathematischen Verfahren wie Korrelation, Registrierung und Ähnlichkeitsanalysen verwendet. Alternativ ist es denkbar, dass das C-Bogen-Röntgengerät geeignete Mittel zum Ermitteln des 2D-Referenz-Bildes aufweist und das C-Bogen-Röntgengerät das 2D-Referenz-Bild als den Eingabeparameter des Bildinformationssystem bereitstellt.

Normalerweise wird nach dem Ermitteln des 2D-Referenz-Bildes das dem 2D-Referenz-Bild zugeordnete zumindest eine 3D-Referenz-Bild als Ausgabe des Bildinformationssystems bestimmt. Üblicherweise ist das 2D-Referenz-Bild bereits dem zumindest einen 3D-Referenz-Bild zugeordnet. Alternativ kann das Zuordnen des 2D-Referenz-Bildes zu dem zumindest einen 3D-Referenz-Bild analog zu dem Bestimmen des zumindest einen 3D-Referenz-Bildes durch das Anwenden des Bildinformationssystems auf das zumindest eine 2D-Bild erfolgen. Insbesondere werden die mathematischen Verfahren wie Korrelation, Registrierung und Ähnlichkeitsanalysen verwendet. In seltenen Fällen kann das Zuordnen des 2D-Referenz-Bildes zu dem zumindest einen 3D-Referenz-Bild durch den Nutzer durch Interaktion mit der graphischen Benutzeroberfläche erfolgen.

Das 2D-Referenz-Bild weist vorzugsweise zumindest einen Teil der Meta-Parameter des zumindest einen 2D-Bildes auf. Beispielsweise kann das 2D-Referenz-Bild mittels des C-Bogen-Röntgengeräts zu einem früheren Zeitpunkt in einer gleichen oder ähnlichen Konfiguration des C-Bogen-Röntgengeräts aufgenommen und dem Bildinformationssystem bereitgestellt worden sein. In diesem Fall weist das 2D-Referenz-Bild beispielsweise die gleiche räumliche Lage des C-Bogen-Röntgengeräts wie beim Erfassen des zumindest einen 2D-Bildes auf. Alternativ ist es denkbar, dass das 2D-Referenz-Bild aus dem 3D-Referenz-Bild beispielsweise mittels der multi-planaren Rekonstruktion berechnet wurde.

Eine Ausführungsform sieht vor, dass das Visualisieren der zumindest einen Kenngröße in dem zumindest einen 2D-Bild umfasst:

- Segmentieren des zumindest einen 2D-Bildes gemäß dem zumindest einen Gefäß und
- Maskieren der zumindest einen Kenngröße in dem zumindest einen segmentierten 2D-Bild.

Vorzugsweise wird die zumindest eine Kenngröße in dem zumindest einen segmentierten 2D-Bild gemäß dem zumindest einen Gefäß maskiert. Das Maskieren der zumindest einen Kenngröße in dem zumindest einen segmentierten 2D-Bild kann vorzugsweise das zumindest eine Gefäß bildlich hervorheben. Der Bereich ohne das zumindest eine Gefäß ist üblicherweise von niedrigem oder keinem Interesse für den Nutzer und wird durch das Maskieren entfernt.

Eine Ausführungsform sieht vor, dass die zumindest eine Kenngröße farbcodiert in dem zumindest einen 2D-Bild visualisiert wird. Wenn die zumindest eine Kenngröße in dem 3D-Referenz-Bild ermittelt wird, wird beispielsweise die zumindest eine Kenngröße in dem 3D-Referenz-Bild farbcodiert und die Farbcodierung der zumindest einen Kenngröße von dem 3D-Referenz-Bild auf das 2D-Referenz-Bild übertragen bzw. projiziert.

Die Farbcodierung der zumindest einen Kenngröße kann das zumindest eine Gefäß in einer ersten Farbe, beispielsweise grün, darstellen, wenn die zumindest eine Kenngröße eher niedrig ist, bzw. in einer zweiten Farbe, beispielsweise rot, darstellen, wenn die zumindest eine Kenngröße eher hoch ist. Das Maß für die Ausprägung der Gefäßverkürzung wird beispielsweise mit einem Winkel zwischen der Mittellinie des zumindest einen Gefäßes und einer Visualisierungsebene des zumindest einen 2D-Bildes in einem Intervall 0-90° ermittelt. Die zumindest eine Kenngröße ist beispielsweise eher in der ersten Farbe visualisiert, wenn der Winkel näher bei 0° liegt, und eher in der zweiten Farbe visualisiert, wenn der Winkel näher bei 90° liegt. Üblicherweise ist die zumindest eine Kenngröße desto mehr in der ersten Farbe visualisiert, je geringer die Gefäßverkürzung ist. Im Gegensatz ist die zumindest eine Kenngröße desto mehr in der zweiten Farbe visualisiert, je höher die Gefäßverkürzung ist.

Die Farbcodierung der zumindest einen Kenngröße gemäß dem zumindest einen 3D-Referenz-Bild kann zusätzlich in dem Bildinformationssystem gespeichert werden. Das Ermitteln der zumindest einen Kenngröße und/oder das Visualisieren der zumindest einen Kenngröße kann daher ein Laden der Farbcodierung der zumindest einen Kenngröße gemäß dem zumindest einen 3D-Referenz-Bildes aus einem Speicher des Bildinformationssystems entsprechen.

Eine Ausführungsform sieht vor, dass das Visualisieren der zumindest einen Kenngröße in dem zumindest einen 2D-Bild eine Projektion der zumindest einen Kenngröße auf das zumindest eine 2D-Bild umfasst.

Die Projektion der zumindest einen Kenngröße erfolgt üblicherweise entlang einer Projektionsrichtung von dem zumindest einen 3D-Referenz-Bild auf das zumindest eine 2D-Bild. Üblicherweise wird zwischen einer Parallel-Projektion und einer perspektivischen Projektion unterschieden. Bei der Parallel-Projektion wird die in dem zumindest einem 3D-Referenz-Bild ermittelte zumindest eine Kenngröße entlang der Projektionsrichtung gemittelt und auf das zumindest eine 2D-Bild übertragen. Die Parallel-Projektion ist eine Idealisierung, weil das zumindest eine 2D-Bild üblicherweise eine perspektivische Abbildung des zumindest einen Gefäßes, insbesondere der Gefäßstruktur oder der Anatomie, aufweist. Die perspektivische Projektion der zumindest einen Kenngröße erfordert typischerweise eine vorherige Registrierung mit optionaler Transformation des zumindest einen 2D-Bildes gemäß dem zumindest einen 3D-Referenz-Bild.

Eine Ausführungsform sieht vor, dass die zumindest eine Kenngröße einer Steuereinheit des C-Bogen-Röntgengeräts bereitgestellt wird, welche gemäß der zumindest einen Kenngröße Steuerbefehle ermittelt, und wobei die Steuereinheit gemäß den Steuerbefehlen das C-Bogen-Röntgengerät zumindest um einen Winkel bewegt und das C-Bogen-Röntgengerät gemäß den Steuerbefehlen ein weiteres 2D-Bild erfasst.

Vorzugweise wird nach dem Ermitteln der zumindest einen Kenngröße in dem zumindest einen 3D-Referenz-Bild das zumindest eine 2D-Bild, in welchem die zumindest eine Kenngröße visualisiert worden ist, dem C-Bogen-Röntgengerät für das Erfassen des weiteren 2D-Bildes bereitgestellt. Alternativ ist es denkbar, dass die ermittelte zumindest einen Kenngröße vor dem Visualisieren dem C-Bogen-Röntgengerät bereitgestellt wird. Vorzugsweise wird in dem weiteren 2D-Bild das zumindest eine Gefäß insbesondere ohne Gefäßverkürzung erfasst. Das zumindest eine Gefäß kann dem Gefäß, beispielsweise einer zu therapierenden Koronararterie, welche in dem weiteren 2D-Bild erfasst werden soll, oder alternativ einem anderen Gefäß entsprechen, welches bereits in dem zumindest einen 2D-Bild abgebildet ist.

Die Steuerbefehle geben insbesondere den zumindest einen Winkel, insbesondere den orbitalen Winkel und/oder den angularen Winkel, des C-Bogen-Röntgengeräts vor. In anderen Worten geben die Steuerbefehle die C-Bogen-Angulation vor. Die Steuereinheit kann das C-Bogen-Röntgengerät gemäß den Steuerbefehlen bewegen. Vorzugsweise können nach dem Bewegen des C-Bogen-Röntgengeräts um den zumindest einen Winkel, insbesondere um den orbitalen Winkel und/oder den angularen Winkel, Messdaten generiert werden, von welchen das weitere 2D-Bild rekonstruiert werden kann. Aus dem zumindest einen 2D-Bild und dem weiteren 2D-Bild kann alternativ oder zusätzlich ein 3D-Bild rekonstruiert werden.

Eine Ausführungsform sieht vor, dass das Bildinformationssystem einen Atlas aufweist, wobei der Atlas zumindest eine Abbildung des zumindest einen 3D-Referenz-Bildes aufweist und wobei durch ein Anwenden des Atlas auf das zumindest eine 2D-Bild das zumindest eine 3D-Referenz-Bild bestimmt wird.

Das Bildinformationssystem, insbesondere der Atlas, weist typischerweise die Bildmenge aus mehreren 3D-Bildern und die weitere 2D-Bildmenge aus mehreren 2D-Bildern von verschiedenen Patienten oder gemäß unterschiedlicher Modelle auf. Die Bildmenge und die weitere 2D-Bildmenge können beispielsweise eine durch ein Bewegungsfeld veränderte Variante des zumindest einen Gefäßes aufweisen. Alternativ können die Bildmenge und die weitere 2D-Bildmenge auch Varianten des zumindest einen Gefäßes aufweisen, welche zu unterschiedlichen Herzphasen oder Atemphasen aufgenommen oder mittels Algorithmen berechnet wurden. Vorzugsweise sind die einzelnen 2D-Bilder bereits jeweils zu den 3D-Bildern zugeordnet. Der Atlas ist vorzugsweise derart ausgestaltet, dass durch ein Anwenden des Atlas auf das zumindest eine 2D-Bild zunächst dasjenige 2D-Bild der 2D-Bildmenge mit der größten Ähnlichkeit ausgewählt wird und das demjenigen 2D-Bild zugeordnete 3D-Bild als das zumindest eine 3D-Referenz-Bild bestimmt wird.

Eine Ausführungsform sieht vor, dass das Bildinformationssystem ein künstliches neuronales Netz aufweist, wobei das künstliche neuronale Netz zum Bestimmen des zumindest einen 3D-Referenz-Bildes durch das Anwenden des künstlichen neuronalen Netzes auf das zumindest eine 2D-Bild trainiert ist und wobei durch ein Anwenden des künstlichen neuronalen Netzes auf das zumindest eine 2D-Bild das zumindest eine 3D-Referenz-Bild bestimmt wird.

Ein künstliches neuronales Netz (KNN, englisch artificial neural network - ANN) ist insbesondere ein in einem Rechenprogramm nachgebildetes Netz aus künstlichen Neuronen. Das künstliche neuronale Netz basiert dabei typischerweise auf einer Vernetzung von mehreren künstlichen Neuronen. Die künstlichen Neuronen sind dabei typischerweise auf verschiedenen Schichten (layers) angeordnet. Üblicherweise umfasst das künstliche neuronale Netz eine Eingangsschicht und eine Ausgabeschicht (output layer), deren Neuronenausgabe als einzige des künstlichen neuronalen Netzes sichtbar wird. Zwischen der Eingangsschicht und der Ausgabeschicht liegende Schichten werden typischerweise als verdeckte Schichten (hidden layer) bezeichnet. Typischerweise wird zunächst eine Architektur und/oder Topologie eines künstlichen neuronalen Netzes initiiert und dann in einer Trainingsphase für eine spezielle Aufgabe oder für mehrere Aufgaben in einer Trainingsphase trainiert. Das Training des künstlichen neuronalen Netzes umfasst dabei typischerweise eine Veränderung einer Gewichtung einer Verbindung zwischen zwei künstlichen Neuronen des künstlichen neuronalen Netzes. Das Training des künstlichen neuronalen Netzes kann auch eine Entwicklung von neuen Verbindungen zwischen künstlichen Neuronen, ein Löschen von bestehenden Verbindungen zwischen künstlichen Neuronen, ein Anpassen von Schwellwerten der künstlichen Neuronen und/oder ein Hinzufügen oder ein Löschen von künstlichen Neuronen umfassen. Zwei unterschiedliche trainierte künstliche neuronale Netze können so, obwohl sie beispielsweise die gleiche Architektur und/oder Topologie aufweisen, unterschiedliche Aufgaben durchführen.

Es wird nun vorgeschlagen, dass ein derart ausgebildetes trainiertes künstliches neuronales Netz gewählt wird, wobei das Anwenden des trainierten künstlichen neuronalen Netzes auf das zumindest eine 2D-Bild ein Bestimmen des zumindest einen 3D-Referenz-Bildes ermöglicht. Das künstliche neuronale Netz kann insbesondere mit der Bildmenge aus mehreren 3D-Bildern und mit der weiteren 2D-Bildmenge aus mehreren 2D-Bildern trainiert worden sein. Das trainierte künstliche neuronale Netz kann dabei lediglich zum Bestimmen des zumindest einen 3D-Referenz-Bildes durch das Anwenden des trainierten künstlichen neuronalen Netzes auf das zumindest eine 2D-Bild geeignet sein. Alternativ kann es auch andere Aufgabe übernehmen. Es kann vorkommen, dass man verschiedene künstliche neuronale Netze aufsetzt, welche das Bestimmen des zumindest einen 3D-Referenz-Bildes gleichermaßen durchführen können.

Das erfindungsgemäße C-Bogen-Röntgengerät umfasst

- eine Planungseinheit,
- eine Steuereinheit und
- eine Messeinheit, welche einen Röntgenstrahler und einen Röntgendetektor aufweist,

wobei das C-Bogen-Röntgengerät dazu ausgebildet ist, ein erfindungsgemäßes Verfahren auszuführen.

Die Planungseinheit kann den Monitor mit der graphischen Benutzeroberfläche und das Eingabegerät aufweisen. Üblicherweise kann der Nutzer mit der Planungseinheit, insbesondere durch das Eingabegerät interagieren. Beispielsweise können dem Nutzer auf dem Monitor C-Bogen-Röntgengerät-Messdaten und/oder das zumindest eine 2D-Bild und/oder das zumindest eine 3D-Referenz-Bild angezeigt werden. Die Planungseinheit kann insbesondere das zumindest eine 2D-Bild, in welchem die zumindest eine Kenngröße visualisiert ist, auf dem Monitor anzeigen.

Die Steuereinheit weist insbesondere eine programmierbare Recheneinheit auf. Insbesondere die Steuereinheit oder die Planungseinheit können das Bildinformationssystem umfassen.

Die Messeinheit weist insbesondere eine Tragevorrichtung für den Röntgenstrahler und den Röntgendetektor auf. Üblicherweise kann die Tragevorrichtung zumindest ein Trageelement, zumindest ein Drehgelenk und/oder zumindest eine Teleskopeinheit aufweisen. Vorzugsweise sind der Röntgenstrahler und der Röntgendetektor an einem C-Bogen, der Teil der Tragevorrichtung ist, angeordnet. Das C-Bogen-Röntgengerät kann vorzugsweise verschiedene räumliche Lagen aufweisen und die verschiedenen räumlichen Lagen gemäß geeigneten Steuerbefehlen automatisch anfahren. Die Steuerbefehle werden vorzugsweise von der Steuereinheit bereitgestellt. Üblicherweise kann die Tragevorrichtung Mittel dafür aufweisen, dass der Röntgenstrahler und der Röntgendetektor gemäß dem orbitalen Winkel oder dem angularen Winkel verfahren werden kann und dass der Abstand zwischen dem Röntgenstrahler und dem Röntgendetektor verändert werden kann.

Das erfindungsgemäße Computerprogrammprodukt ist direkt in einen Speicher einer programmierbaren Recheneinheit ladbar und weist Programmcode-Mittel auf, um ein erfindungsgemäßes Verfahren auszuführen, wenn das Computerprogrammprodukt in der Recheneinheit ausgeführt wird.

Das Computerprogrammprodukt kann ein Computerprogramm sein oder ein Computerprogramm umfassen. Dadurch kann das erfindungsgemäße Verfahren schnell, identisch wiederholbar und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk oder Server hinterlegt, von wo es in den Prozessor der Recheneinheit geladen werden kann, der beispielsweise als Teil des C-Bogen-Röntgengeräts ausgebildet sein kann. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit ein erfindungsgemäßes Verfahren ausführen. So kann das Computerprogrammprodukt auch den elektronisch lesbaren Datenträger darstellen. Beispiele für elektronisch lesbare Datenträger sind eine DVD, ein Magnetband, eine Festplatte oder ein USB-Stick, auf welchem elektronisch lesbare Steuerinformationen, insbesondere Software (vgl. oben), gespeichert ist. Wenn diese Steuerinformationen (Software) von dem Datenträger gelesen und in der Recheneinheit und/oder Planungseinheit und/oder Messeinheit des C-Bogen-Röntgengeräts gespeichert werden, können alle erfindungsgemäßen Ausführungsformen der vorab beschriebenen Verfahren durchgeführt werden. So kann die Erfindung auch von dem besagten computerlesbaren Medium und/oder dem besagten elektronisch lesbaren Datenträger ausgehen.

Im Folgenden wird die Erfindung anhand der in den Figuren dargestellten Ausführungsbeispiele näher beschrieben und erläutert.

Es zeigen:

Fig. 1 ein erfindungsgemäßes C-Bogen-Röntgengerät,

Fig. 2 ein Flussdiagramm des erfindungsgemäßen Verfahrens und

Fig. 3 eine Ausführungsform des erfindungsgemäßen Verfahrens.

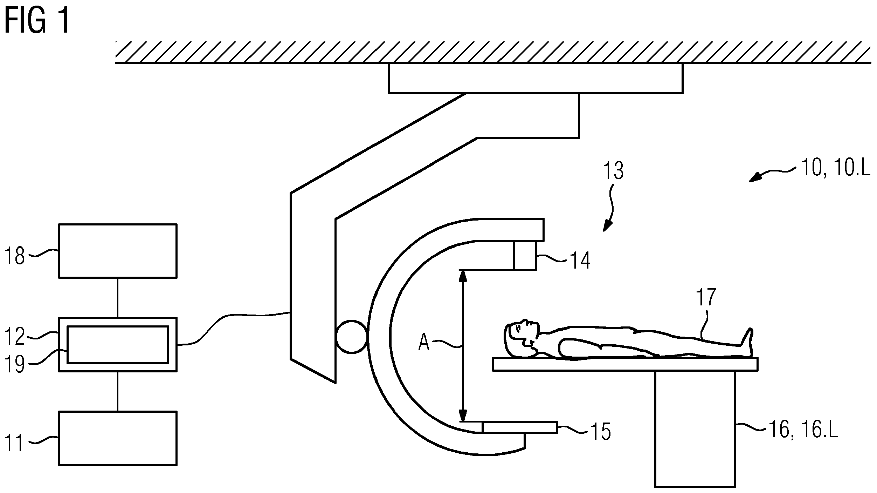

Fig. 1 zeigt ein erfindungsgemäßes C-Bogen-Röntgengerät 10, welches eine Planungseinheit 11, eine Steuereinheit 12 und eine Messeinheit 13 umfasst. Die Messeinheit 13 umfasst einen Röntgenstrahler 14 und einen Röntgendetektor 15. Die Steuereinheit 12 weist insbesondere eine programmierbare Recheneinheit 19 auf. Auf einem höhenverstellbaren und zusätzlich in zwei Raumrichtungen verfahrbaren Patientientisch 16 ist der Patient 17 angeordnet. Im gezeigten Fall ist das Bildinformationssystem 18 als Teil des C-Bogen-Röntgengeräts 10 ausgebildet. Alternativ ist es auch denkbar, dass das Bildinformationssystem 18 in einem Rechenzentrum bereitgestellt ist und mittels einer geeigneten Schnittstelle mit dem C-Bogen-Röntgengerät 10 verbunden ist. In diesem Fall wird ein System aus dem Bildinformationssystem 18 und dem Röntgengerät 10 beansprucht.

Das Bildinformationssystem 18 ist hinsichtlich eines Datenaustauschs mit der Steuereinheit 12, insbesondere der programmierbaren Recheneinheit 19, verbunden.

Das Bildinformationssystem 18 weist einen Atlas auf, wobei der Atlas zumindest eine Abbildung des zumindest einen 3D-Referenz-Bildes aufweist und wobei durch ein Anwenden des Atlas auf das zumindest eine 2D-Bild das zumindest eine 3D-Referenz-Bild bestimmt wird.

Gemäß einer alternativen, nicht dargestellten, Ausführung weist das Bildinformationssystem 18 ein künstliches neuronales Netz auf, wobei das künstliche neuronale Netz zum Bestimmen des zumindest einen 3D-Referenz-Bildes durch das Anwenden des künstlichen neuronalen Netzes auf das zumindest eine 2D-Bild trainiert ist und wobei durch ein Anwenden des künstlichen neuronalen Netzes auf das zumindest eine 2D-Bild das zumindest eine 3D-Referenz-Bild bestimmt wird.

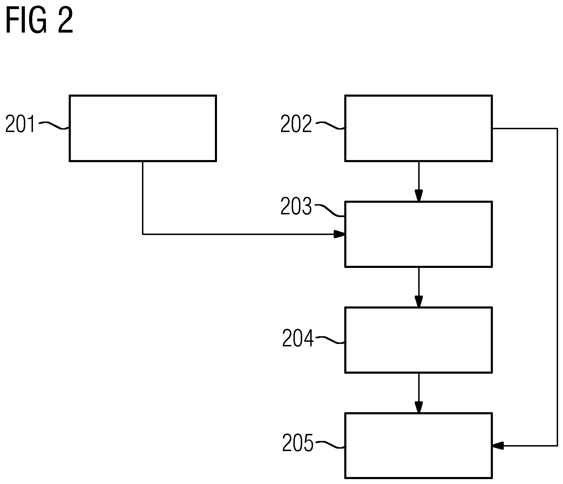

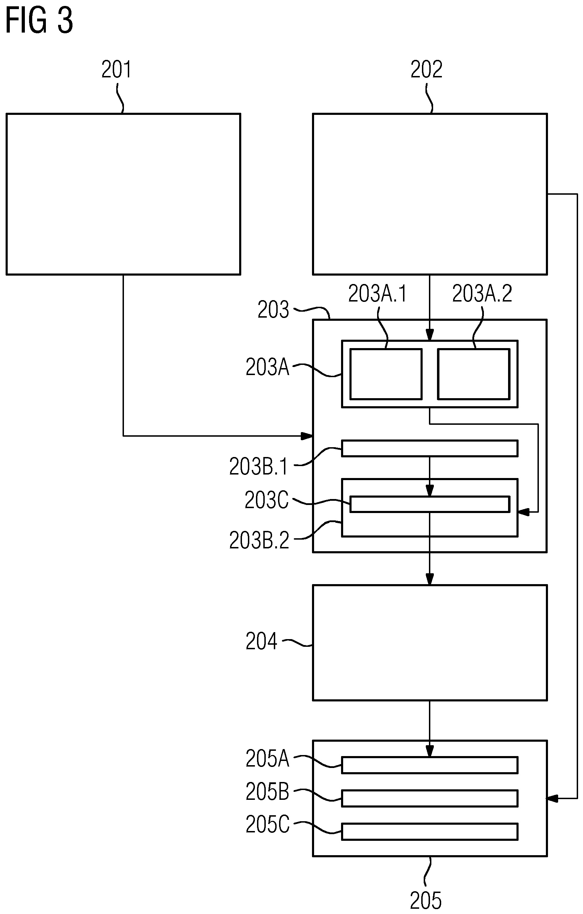

Das C-Bogen-Röntgengerät 10 ist, insbesondere mit der Planungseinheit 11, der Steuereinheit 12 und Messeinheit 13, dazu ausgebildet, ein Verfahren gemäß Fig. 2 oder Fig. 3 auszuführen.

Fig. 2 zeigt ein Flussdiagramm des erfindungsgemäßen Verfahrens zum Visualisieren zumindest einer Kenngröße des Patienten 17 bei einer angiographischen Messung mittels des C-Bogen-Röntgengeräts 10. Das erfindungsgemäße Verfahren umfasst die Verfahrensschritte 201-205.

Verfahrensschritt 201 kennzeichnet das Bereitstellen eines Bildinformationssystems 18.

Verfahrensschritt 202 kennzeichnet das Erfassen zumindest eines 2D-Bildes des Patienten 17 mittels des C-Bogen-Röntgengeräts 10.

Verfahrensschritt 203 kennzeichnet das Bestimmen zumindest eines 3D-Referenz-Bildes durch Anwenden des Bildinformationssystems 18 auf das zumindest eine 2D-Bild.

Verfahrensschritt 204 kennzeichnet das Ermitteln zumindest einer Kenngröße, welche zumindest ein Gefäß des Patienten 17 beschreibt, in dem zumindest einen 3D-Referenz-Bild.

Verfahrensschritt 205 kennzeichnet das Visualisieren der zumindest einen Kenngröße in dem zumindest einen 2D-Bild. Beispielsweise kann das C-Bogen-Röntgengerät das zumindest eine 2D-Bild, in welchem die zumindest eine Kenngröße visualisiert worden ist, auf einem Monitor der Planungseinheit 11 anzeigen. Alternativ oder zusätzlich kann das Bildinformationssystem 18 einen weiteren Monitor aufweisen, auf welchem das zumindest eine 2D-Bild, in welchem die zumindest eine Kenngröße visualisiert worden ist, angezeigt werden kann.

Fig. 3 zeigt eine Ausführungsform des erfindungsgemäßen Verfahrens zum Visualisieren zumindest einer Kenngröße des Patienten 17 bei einer angiographischen Messung mittels des C-Bogen-Röntgengeräts 10.

Die nachfolgende Beschreibung beschränkt sich im Wesentlichen auf die Unterschiede zu dem Ausführungsbeispiel in Fig. 2, wobei bezüglich gleich bleibender Verfahrensschritte auf die Beschreibung des Ausführungsbeispiels in Fig. 2 verwiesen wird. Im Wesentlichen gleich bleibende Verfahrensschritte sind grundsätzlich mit den gleichen Bezugszeichen beziffert.

Die zumindest eine Kenngröße beschreibt eine Gefäßverkürzung des zumindest einen Gefäßes. Alternativ oder zusätzlich kann die zumindest eine Kenngröße eine Gefäßüberlappung des zumindest einen Gefäßes beschreiben.

Verfahrensschritt 203A kennzeichnet, dass dem zumindest einen 2D-Bild zumindest ein Meta-Parameter zugeordnet wird, wobei der zumindest eine Meta-Parameter beim Bestimmen des zumindest einen 3D-Referenz-Bildes verwendet wird und der zumindest eine Meta-Parameter mindestens einen Parameter der folgenden Liste aufweist:

- eine Meta-Information über das Erfassen des zumindest einen 2D-Bildes mittels des C-Bogen-Röntgengeräts,

- eine Gefäßbezeichnung des zumindest einen Gefäßes und

- eine Herzphase des Patienten 17.

Verfahrensschritt 203A.1 kennzeichnet, dass der zumindest eine Meta-Parameter die Meta-Information über das Erfassen des zumindest einen 2D-Bildes mittels des C-Bogen-Röntgengeräts 10 aufweist und dass die Meta-Information mindestens einen Parameter der folgenden Liste aufweist:

- eine räumliche Lage 10.L des C-Bogen-Röntgengeräts 10,
- einen Abstand A zwischen einem Röntgenstrahler 14 des C-Bogen-Röntgengeräts 10 und einem Röntgendetektor 15 des C-Bogen-Röntgengeräts 10 und
- eine räumliche Lage 16.L des Patiententisches 16.

Die räumliche Lage 10.L, der Abstand A und die räumliche Lage 16.L können jeweils über geeignete Transformationen, insbesondere Koordinatentransformationen, zu einander in Relation gesetzt werden. Beispielsweise kann das zumindest eine 2D-Bild und/oder das zumindest eine 3D-Referenz-Bild die räumliche Lage 10.L, den Abstand A und die räumliche Lage 16.L als Teil des jeweiligen Headers aufweisen.

Verfahrensschritt 203A.2 kennzeichnet, dass der zumindest eine Meta-Parameter die Gefäßbezeichnung des zumindest einen Gefäßes aufweist und dass die Gefäßbezeichnung mindestens einen Parameter der folgenden Liste aufweist:

- einen Gefäßdurchmesser des zumindest einen Gefäßes,
- eine maximale Krümmung des zumindest einen Gefäßes und
- eine Länge des zumindest einen Gefäßes.

Verfahrensschritt 203B.1 kennzeichnet, dass das Bestimmen des zumindest einen 3D-Referenz-Bildes durch das Anwenden des Bildinformationssystems 18 auf das zumindest eine 2D-Bild ein Segmentieren des zumindest einen 2D-Bildes gemäß dem zumindest einen Gefäß umfasst.

Verfahrensschritt 203B.2 kennzeichnet, dass das Bestimmen des zumindest einen 3D-Referenz-Bildes durch das Anwenden des Bildinformationssystems 18 auf das zumindest eine 2D-Bild ein Anwenden des Bildinformationssystems 18 auf das zumindest eine segmentierte 2D-Bild umfasst.

In den Verfahrensschritt 203B.2 geht das zumindest eine segmentierte 2D-Bild aus dem Verfahrensschritt 203B.1 und der zumindest eine Meta-Parameter aus dem Verfahrensschritt 203A ein.

Verfahrensschritt 203C kennzeichnet, dass das Bestimmen des zumindest einen 3D-Referenz-Bildes durch das Anwenden des Bildinformationssystems 18 auf das zumindest eine 2D-Bild das Ermitteln eines dem zumindest einen 2D-Bild zugeordneten 2D-Referenz-Bildes umfasst, wobei das 2D-Referenz-Bild dem zumindest einen 3D-Referenz-Bild zugeordnet ist.

Grundsätzlich ist es denkbar, dass die Verfahrensschritte 203, 203A, 203A.1, 203A.2, 203B.1, 203B.2 und 203C lediglich separat voneinander oder in einer beliebigen Kombination dieser Verfahrensschritte durchgeführt werden.

Verfahrensschritt 205A kennzeichnet, dass die zumindest eine Kenngröße farbcodiert in dem zumindest einen 2D-Bild visualisiert wird. Die Farbcodierung im RGB-Farbraum (RGB = rot, grün, blau) kann beispielsweise wie folgt berechnet werden:

$$Rot = \cos(90° - alpha)$$

$$Grün = 1 - Rot$$

$$Blau = 0$$

Die Parameter Rot, Grün und Blau geben die jeweiligen Farbanteile wieder. Der Parameter alpha entspricht einem Winkel zwischen dem zumindest einen Gefäßes, insbesondere einer Mittellinie des zumindest einen Gefäßes, und der Visualisierungsebene des zumindest einen 2D-Bildes im Intervall 0-90°.

Eine weitere alternative Formel zur Farbcodierung lautet:

$$Rot = \sqrt{Normalisierungswert}$$

$$Grün = 1 - Rot$$

$$Blau = 0$$

Der Normalisierungswert wird berechnet gemäß dem absoluten Winkel der Mittellinie ohne Vorzeichen, beispielsweise ist der Normalisierungswert 0, wenn der absolute Winkel 0° ist, und der Normalisierungswert ist 1, wenn der absolute Winkel 90° ist.

Verfahrensschritt 205B kennzeichnet, dass das Visualisieren der zumindest einen Kenngröße in dem zumindest einen 2D-Bild eine Projektion der zumindest einen Kenngröße auf das zumindest eine 2D-Bild umfasst.

Verfahrensschritt 205C kennzeichnet, dass das das Visualisieren der zumindest einen Kenngröße in dem zumindest einen 2D-Bild umfasst:

- Segmentieren des zumindest einen 2D-Bildes gemäß dem zumindest einen Gefäß und
- Maskieren der zumindest einen Kenngröße in dem zumindest einen segmentierten 2D-Bild.

Insbesondere die Verfahrensschritte 204 und 205 sowie die untergeordneten Verfahrensschritte 205A, 205B und 205C können sowohl mittels des C-Bogen-Röntgengeräts 10 als auch mittels des Bildinformationssystem 18 durchgeführt werden. Eine getrennte Durchführung der genannten Verfahrensschritte ist insofern denkbar, wenn das Bildinformationssystem 18 beispielsweise in einem Rechenzentrum außerhalb eines Krankenhauses vorliegt, während sich das C-Bogen-Röntgengengerät in dem Krankenhaus befindet.

Gemäß einer ersten Variante werden die Verfahrensschritte 204 und 205 sowie die untergeordneten Verfahrensschritte 205A, 205B und 205C mittels des C-Bogen-Röntgengeräts 10 durchgeführt.

Gemäß einer zweiten Variante wird der Verfahrensschritt 204 mittels des Bildinformationssystem 18 und der Verfahrensschritt 205 sowie die untergeordneten Verfahrensschritte 205A, 205B und 205C mittels des C-Bogen-Röntgengeräts 10 durchgeführt.

Gemäß einer dritten Variante werden die Verfahrensschritte 204 und 205 sowie die untergeordneten Verfahrensschritte 205A, 205B und 205C mittels des Bildinformationssystems 18 durchgeführt.

In einer alternativen, nicht gezeigten Ausführung können die Verfahrensschritte 205A, 205B und 205C beliebig kombiniert sowie separat voneinander durchgeführt werden.

Dem Fachmann sind unter Umständen weitere Möglichkeiten über die hier gezeigten drei Varianten hinaus zu einer erfindungsgemäßen Durchführung des Verfahrens bekannt, welche aus Gründen der Übersichtlichkeit hier nicht gezeigt werden.

Das C-Bogen-Röntgengerät 10 und das Bildinformationssystem 18 weisen daher insbesondere Mittel zu einem Senden, einem Empfangen und einem Anzeigen des zumindest einen 2D-Bildes, des zumindest einen 3D-Referenz-Bildes, des 2D-Referenz-Bildes und/oder des zumindest einen 2D-Bildes, in welchem die zumindest eine Kenngröße visualisiert worden ist, auf. Vorzugsweise erfolgt das Senden, Empfangen und/oder Anzeigen automatisch.

Das zumindest eine 2D-Bild, das zumindest eine 3D-Referenz-Bild, das 2D-Referenz-Bild und/oder das zumindest eine 2D-Bild, in welchem die zumindest eine Kenngröße visualisiert worden ist, können in einem geeigneten Format gespeichert werden und/oder gemäß eines geeigneten Netzwerk-Protokolls übertragen werden.

Gemäß einer weiteren Ausführung wird die zumindest eine Kenngröße einer Steuereinheit 12 des C-Bogen-Röntgengeräts 10 bereitgestellt, welche gemäß der zumindest einen Kenngröße Steuerbefehle ermittelt, und die Steuereinheit 12 bewegt gemäß den Steuerbefehlen das C-Bogen-Röntgengerät 10 zumindest um einen Winkel und das C-Bogen-Röntgengerät 10 erfasst gemäß den Steuerbefehlen ein weiteres 2D-Bild.

Die in Fig. 2 und in Fig. 3 dargestellten Verfahrensschritte des erfindungsgemäßen Verfahrens werden von der Recheneinheit 19 des C-Bogen-Röntgengeräts 10 ausgeführt. Hierzu umfasst die Recheneinheit 19 erforderliche Software und/oder Computerprogramme und/oder ein Computerprogrammprodukt, die in einer Speichereinheit der Recheneinheit 19 gespeichert sind. Die Software und/oder Computerprogramme und/oder das Computerprogrammprodukt umfassen Programmmittel, die dazu ausgelegt sind, die dargestellten Verfahrensschritte auszuführen, wenn das Computerprogramm und/oder die Software und/oder das Computerprogrammprodukt in der Recheneinheit 19 mittels einer Prozessoreinheit der Recheneinheit 19 ausgeführt wird.

Obwohl die Erfindung im Detail durch das bevorzugte Ausführungsbeispiel näher illustriert und beschrieben wurde, ist die Erfindung nicht durch die offenbarten Beispiele eingeschränkt. Variationen hiervon können vom Fachmann abgeleitet werden, ohne den Schutzumfang der Erfindung, wie er durch die nachfolgenden Patentansprüche definiert wird, zu verlassen.

**Patentansprüche**

1.  Verfahren zum Visualisieren zumindest einer Kenngröße eines Patienten bei einer angiographischen Messung mittels eines C-Bogen-Röntgengeräts, umfassend folgende Schritte:

    - Bereitstellen (201) eines Bildinformationssystems (18),
    - Erfassen (202) zumindest eines 2D-Bildes des Patienten (17) mittels des C-Bogen-Röntgengeräts (10),
    - Bestimmen (203) zumindest eines 3D-Referenz-Bildes durch Anwenden des Bildinformationssystems (18) auf das zumindest eine 2D-Bild,
    - Ermitteln (204) zumindest einer Kenngröße, welche zumindest ein Gefäß des Patienten (17) beschreibt, in dem zumindest einen 3D-Referenz-Bild und
    - Visualisieren (205) der zumindest einen Kenngröße in dem zumindest einen 2D-Bild.

2.  Verfahren nach Anspruch 1, wobei die zumindest eine Kenngröße eine Gefäßverkürzung des zumindest einen Gefäßes beschreibt.

3.  Verfahren nach einem der vorhergehenden Ansprüche, wobei die zumindest eine Kenngröße eine Gefäßüberlappung des zumindest einen Gefäßes beschreibt.

4.  Verfahren nach einem der vorhergehenden Ansprüche, wobei dem zumindest einen 2D-Bild zumindest ein Meta-Parameter zugeordnet ist, wobei der zumindest eine Meta-Parameter beim Bestimmen des zumindest einen 3D-Referenz-Bildes verwendet wird und der zumindest eine Meta-Parameter mindestens einen Parameter der folgenden Liste aufweist:

    - eine Meta-Information über das Erfassen des zumindest einen 2D-Bildes mittels des C-Bogen-Röntgengeräts,
- eine Gefäßbezeichnung des zumindest einen Gefäßes und
- eine Herzphase des Patienten.

5. Verfahren nach Anspruch 4, wobei der zumindest eine Meta-Parameter die Meta-Information über das Erfassen des zumindest einen 2D-Bildes mittels des C-Bogen-Röntgengeräts aufweist und wobei die Meta-Information mindestens einen Parameter der folgenden Liste aufweist:

- eine räumliche Lage (10.L) des C-Bogen-Röntgengeräts (10),
- einen Abstand (A) zwischen einem Röntgenstrahler (14) des C-Bogen-Röntgengeräts (10) und einem Röntgendetektor (15) des C-Bogen-Röntgengeräts (10) und
- eine räumliche Lage (16.L) des Patiententisches (16).

6. Verfahren nach einem der Ansprüche 4 bis 5, wobei der zumindest eine Meta-Parameter die Gefäßbezeichnung des zumindest einen Gefäßes aufweist und wobei die Gefäßbezeichnung mindestens einen Parameter der folgenden Liste aufweist:

- einen Gefäßdurchmesser des zumindest einen Gefäßes,
- eine maximale Krümmung des zumindest einen Gefäßes und
- eine Länge des zumindest einen Gefäßes.

7. Verfahren nach einem der vorhergehenden Ansprüche, wobei das Bestimmen des zumindest einen 3D-Referenz-Bildes durch das Anwenden des Bildinformationssystems auf das zumindest eine 2D-Bild umfasst:

- Segmentieren des zumindest einen 2D-Bildes gemäß dem zumindest einen Gefäß und
- Anwenden des Bildinformationssystems auf das zumindest eine segmentierte 2D-Bild.

8. Verfahren nach einem der vorhergehenden Ansprüche, wobei das Bestimmen des zumindest einen 3D-Referenz-Bildes durch das Anwenden des Bildinformationssystems auf das zumindest eine 2D-Bild ein Ermitteln eines 2D-Referenz-Bildes, wobei das 2D-Referenz-Bild dem zumindest einen 3D-Referenz-Bild zugeordnet ist, umfasst.

9. Verfahren nach einem der vorhergehenden Ansprüche, wobei das Visualisieren der zumindest einen Kenngröße in dem zumindest einen 2D-Bild umfasst:

- Segmentieren des zumindest einen 2D-Bildes gemäß dem zumindest einen Gefäß und
- Maskieren der zumindest einen Kenngröße in dem zumindest einen segmentierten 2D-Bild.

10. Verfahren nach einem der vorhergehenden Ansprüche, wobei die zumindest eine Kenngröße farbcodiert in dem zumindest einen 2D-Bild visualisiert wird.

11. Verfahren nach einem der vorhergehenden Ansprüche, wobei das Visualisieren der zumindest einen Kenngröße in dem zumindest einen 2D-Bild eine Projektion der zumindest einen Kenngröße auf das zumindest eine 2D-Bild umfasst.

12. Verfahren nach einem der vorhergehenden Ansprüche, wobei die zumindest eine Kenngröße einer Steuereinheit des C-Bogen-Röntgengeräts bereitgestellt wird, welche gemäß der zumindest einen Kenngröße Steuerbefehle ermittelt, und wobei die Steuereinheit gemäß den Steuerbefehlen das C-Bogen-Röntgengerät zumindest um einen Winkel bewegt und das C-Bogen-Röntgengerät gemäß den Steuerbefehlen ein weiteres 2D-Bild erfasst.

13. Verfahren nach einem der vorhergehenden Ansprüche, wobei das Bildinformationssystem einen Atlas aufweist, wobei der Atlas zumindest eine Abbildung des zumindest einen 3D-Referenz-Bildes aufweist und wobei durch ein Anwenden des Atlas auf das zumindest eine 2D-Bild das zumindest eine 3D-Referenz-Bild bestimmt wird.

14. Verfahren nach einem der vorhergehenden Ansprüche, wobei das Bildinformationssystem ein künstliches neuronales Netz aufweist, wobei das künstliche neuronale Netz zum Bestimmen des zumindest einen 3D-Referenz-Bildes durch das Anwenden des künstlichen neuronalen Netzes auf das zumindest eine 2D-Bild trainiert ist und wobei durch ein Anwenden des künstlichen neuronalen Netzes auf das zumindest eine 2D-Bild das zumindest eine 3D-Referenz-Bild bestimmt wird.

15. C-Bogen-Röntgengerät, umfassend

- eine Planungseinheit,
- eine Steuereinheit und
- eine Messeinheit, welche einen Röntgenstrahler und einen Röntgendetektor aufweist,

wobei das C-Bogen-Röntgengerät dazu ausgebildet ist, ein Verfahren nach einem der vorhergehenden Ansprüche auszuführen.

16. Computerprogrammprodukt, welches direkt in einen Speicher einer programmierbaren Recheneinheit ladbar ist, mit Programmcode-Mitteln, um ein Verfahren nach einem der Ansprüche 1 bis 14 auszuführen, wenn das Computerprogrammprodukt in der Recheneinheit ausgeführt wird.

**Claims**

1. Method for visualising at least one characteristic variable of a patient in an angiographic scan by means of a C-arm X-ray device comprising the following steps:

    - providing (201) an image information system (18),
    - acquiring (202) at least one 2-D image of the patient (17) by means of the C-arm X-ray device (10),
    - determining (203) at least one 3-D reference image by applying the image information system (18) to the at least one 2-D image,
    - establishing (204) at least one characteristic variable which describes at least one vessel of the patient (17) in the at least one 3-D reference image, and
    - visualising (205) the at least one characteristic variable in the at least one 2-D image.

2. Method according to claim 1, wherein the at least one characteristic variable describes a vessel foreshortening of the at least one vessel.

3. Method according to one of the preceding claims, wherein the at least one characteristic variable describes a vessel overlap of the at least one vessel.

4. Method according to one of the preceding claims wherein at least one metaparameter is assigned to the at least one 2-D image, wherein the at least one metaparameter is used on determining the at least one 3-D reference image and the at least one metaparameter comprises at least one parameter of the following list:

    - an item of metainformation regarding the acquisition of the at least one 2-D image by means of the C-arm X-ray device,
    - a vessel designation of the at least one vessel, and
    - a cardiac phase of the patient.

5. Method according to claim 4, wherein the at least one metaparameter comprises the metainformation regarding the acquisition of the at least one 2-D image by means of the C-arm X-ray device and wherein the metainformation comprises at least one parameter of the following list:

    - a spatial position (10.L) of the C-arm X-ray device (10),
    - a spacing (A) between an X-ray radiator (14) of the C-arm X-ray device (10) and an X-ray detector (15) of the C-arm X-ray device (10), and
    - a spatial position (16.L) of the patient table (16).

6. Method according to one of claims 4 to 5, wherein the at least one metaparameter comprises the vessel designation of the at least one vessel and wherein the vessel designation comprises at least one parameter of the following list:

    - a vessel diameter of the at least one vessel,
    - a maximum curvature of the at least one vessel, and
    - a length of the at least one vessel.

7. Method according to one of the preceding claims, wherein the determination of the at least one 3-D reference image by the application of the image information system to the at least one 2-D image comprises:

    - segmenting the at least one 2-D image according to the at least one vessel, and
    - applying the image information system to the at least one segmented 2-D image.

8. Method according to one of the preceding claims, wherein the determination of the at least one 3-D reference image by the application of the image information system to the at least one 2-D image comprises an establishment of a 2-D reference image, wherein the 2-D reference image is assigned to the at least one 3-D reference image.

9. Method according to one of the preceding claims, wherein the visualisation of the at least one characteristic variable in the at least one 2-D image comprises:

    - segmenting the at least one 2-D image according to the at least one vessel, and
    - masking the at least one characteristic variable in the at least one segmented 2-D image.

10. Method according to one of the preceding claims, wherein the at least one characteristic variable is visualised in a colour-coded manner in the at least one 2-D image.

11. Method according to one of the preceding claims, wherein the visualisation of the at least one characteristic variable in the at least one 2-D image comprises a projection of the at least one characteristic variable onto the at least one 2-D image.

12. Method according to one of the preceding claims, wherein the at least one characteristic variable of a control unit of the C-arm X-ray device is provided which according to the at least one characteristic variable establishes control commands and wherein the control unit moves the C-arm X-ray device in accordance with the control commands at least through an angle and the C-arm X-ray device acquires a further 2-D image in accordance with the control commands.

13. Method according to one of the preceding claims, the image information system comprising an atlas, wherein the atlas comprises at least one depiction of the at least one 3-D reference image and wherein by an application of the atlas to the at least one 2-D image, the at least one 3-D reference image is determined.

14. Method according to one of the preceding claims, wherein the image information system comprises an artificial neural network, wherein the artificial neural network is trained for determining the at least one 3-D reference image through the application of the artificial neural network to the at least one 2-D image and wherein by an application of the artificial neural network to the at least one 2-D image, the at least one 3-D reference image is determined.

15. C-arm X-ray device, comprising

   - a planning unit,
   - a control unit, and
   - a scanning unit which comprises an X-ray radiator and an X-ray detector,

   wherein the C-arm X-ray device is configured to carry out a method according to one of the preceding claims.

16. Computer program product which is directly loadable into a memory store of a programmable computer unit, having program code means in order to carry out a method according to one of claims 1 to 14 when the computer program product is executed in the computer unit.

**Revendications**

1. Procédé de visualisation d'au moins une grandeur caractéristique d'un patient dans une mesure angiographique au moyen d'un appareil radiologique à arc en C, comprenant les stades suivants :

   - on se procure (201) un système (18) d'information d'image,
   - on prend (202) au moins une image en 2D du patient (17), au moyen de l'appareil (10) radiologique à arc en C,
   - on détermine (203) au moins une image de référence en 3D, en appliquant le système (18) d'information d'image à la au moins une image en 2D,
   - on détermine (204) au moins une grandeur caractéristique, qui décrit au moins un vaisseau du patient (17) dans la au moins une image de référence en 3D et
   - on visualise (205) la au moins une grandeur caractéristique dans la au moins une image en 2D.

2. Procédé suivant la revendication 1, dans lequel la au moins une grandeur caractéristique décrit un raccourcissement dudit au moins un vaisseau.

3. Procédé suivant l'une des revendications précédentes, dans lequel la au moins une grandeur caractéristique décrit un chevauchement du au moins un vaisseau.

4. Procédé suivant l'une des revendications précédentes, dans lequel on associe au moins un méta-paramètre à la au moins une image en 2D, le au moins un méta-paramètre étant utilisé lors de la détermination de la au moins une image de référence en 3D et le au moins un méta-paramètre ayant au moins un paramètre de la liste suivante :

   - une méta-information sur la détection de la au moins une image en 2D au moyen de l'appareil radiologique à arc en C,
   - une désignation du au moins un vaisseau et
   - une face cardiaque du patient.

5. Procédé suivant la revendication 4, dans lequel le au moins un méta-paramètre a la méta-information sur la détection de la au moins une image en 2D au moyen de l'appareil radiologique à arc en C et dans lequel la méta-information a au moins un paramètre de la liste suivante :

   - une position (10.L) dans l'espace de l'appareil (10) radiologique à arc en C,
   - une distance (A) entre un émetteur (14) de rayons X de l'appareil (10) radiologique à arc en C et un détecteur (15) de rayons X de l'appareil (10) radiologique à arc en C et
   - une position (16.L) dans l'espace de la couchette (16) du patient.

6. Procédé suivant l'une des revendications 4 à 5, dans lequel le au moins un méta-paramètre a la désignation du au moins un vaisseau et dans lequel la désignation du vaisseau contient au moins un paramètre de la liste suivant :

- un diamètre du au moins un vaisseau,
- une courbure maximum du au moins un vaisseau et
- une longueur du au moins un vaisseau.

7. Procédé suivant au moins l'une des revendications précédentes, dans lequel la détermination de la au moins une image de référence en 3D, par l'application du système d'information d'image à la au moins une image en 2D, comprend :

   - la segmentation de la au moins une image en 2D suivant le au moins un vaisseau et
   - l'application du système d'information d'image à la au moins une image en 2D segmentée.

8. Procédé suivant l'une des revendications précédentes, dans lequel la détermination de la au moins une image de référence en 3D, par l'application du système d'information d'image à la au moins une image en 2D, comprend une détermination d'une image de référence en 2D, l'image de référence en 2D étant associée à la au moins une image de référence en 3D.

9. Procédé suivant l'une des revendications précédentes, dans lequel la visualisation de la au moins une grandeur caractéristique dans la au moins une image en 2D comprend :

   - la segmentation de la au moins une image en 2D suivant le au moins un vaisseau et
   - le masquage de la au moins une grandeur caractéristique dans la au moins une image en 2D segmentée.

10. Procédé suivant l'une des revendications précédentes, dans lequel on visualise la au moins une grandeur caractéristique d'une manière codée en couleur dans la au moins une image en 2D.

11. Procédé suivant l'une des revendications précédentes, dans lequel la visualisation de la au moins une grandeur caractéristique dans la au moins une image en 2D comprend une projection de la au moins une grandeur caractéristique sur la au moins une image en 2D.

12. Procédé suivant l'une des revendications précédentes, dans lequel on met la au moins une grandeur caractéristique à disposition d'une unité de commande de l'appareil radiologique à arc en C, qui détermine, suivant la au moins une grandeur caractéristique, des instructions de commande et dans lequel l'unité de commande déplace, suivant les instructions de commande, au moins d'un angle l'appareil radiologique à arc en C et l'appareil radiologique à arc en C prend, suivant les instructions de commande, une autre image en 2D.

13. Procédé suivant l'une des revendications précédentes, dans lequel le système d'information d'image a un atlas, l'atlas ayant au moins une reproduction de la au moins une image de référence en 3D et la au moins une image de référence en 3D étant déterminée par une application de l'atlas à la au moins une image en 2D.

14. Procédé suivant l'une des revendications précédentes, dans lequel le système d'information d'image a un réseau neuronal artificiel, le réseau neuronal artificiel ayant subi un apprentissage pour déterminer la au moins une image de référence en 3D par l'application du réseau neuronal artificiel à la au moins une image en 2D et dans lequel on détermine la au moins une image de référence en 3D en appliquant le réseau neuronal artificiel à la au moins une image en 2D.

15. Appareil radiologique à arc en C, comprenant

   - une unité de conception,
   - une unité de commande et
   - une unité de mesure, qui a un émetteur de rayons X et un détecteur de rayons X,

dans lequel l'appareil radiologique à arc en C est constitué pour exécuter un procédé suivant l'une des revendications précédentes.

16. Produit de programme d'ordinateur, qui peut être chargé directement dans une mémoire d'une unité d'ordinateur programmable, comprenant des moyens de code de programme pour exécuter un procédé suivant l'une des revendications 1 à 14, lorsque le produit de programme d'ordinateur est réalisé dans l'unité d'ordinateur.

FIG 1

EP 3 420 903 B1

FIG 2

201 ⌐▭

202 ⌐▭

203 ⌐▭

204 ⌐▭

205 ⌐▭

# FIG 3

201    202

203    203A.1    203A.2

203A

203B.1

203C

203B.2

204

205A

205B

205C

205

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015202082 A1
- DE 102014202013 A1
- DE 102012208850 A1
- EP 3128481 A